# EUROPEAN PATENT SPECIFICATION

(11) **EP 2 742 355 B1**
(45) Date of publication and mention of the grant of the patent: **19.12.2018**
(21) Application number: 12824498.5
(22) Date of filing: 13.08.2012
(51) Int. Cl.: G01N 33/564

(54) **METHOD OF DIAGNOSING BACTERIAL INFECTIONS USING BACTERIAL GLYCOPROTEINS**
VERFAHREN ZUR DIAGNOSE VON BAKTERIELLEN INFEKTIONEN MIT BAKTERIELLEN GLYCOPROTEINEN
PROCÉDÉ DE DIAGNOSTIC D'INFECTIONS BACTÉRIENNES UTILISANT DES GLYCOPROTÉINES BACTÉRIENNES

(30) Priority: 12.08.2011 US 201161522967 P
(43) Date of publication of application: 18.06.2014
(73) Proprietor: The Governors of the University of Alberta, Edmonton, Alberta T5J 4P6 (CA); Instituto De Investigaciones Biotecnologicas- Instituto Tecnologico De Chascomus Consejo De Investigaciones Cientificas y Tecnicas, Buenos Aires (AR)
(72) Inventor: FELDMAN, Mario, Edmonton, Alberta T6R 3R1 (CA); IWASHKIW, Jeremy, Edmonton, Alberta T6E 4W4 (CA); UGALDE, Juan Esteban, Buenos Aires 1431 (AR); CIOCCHINI, Andrés Eduardo, Buenos Aires 1425 (AR); COMERCI, Diego José, Buenos Aires 1602 (AR)
(74) Representative: Appleyard Lees IP LLP
(86) International application number: PCT/CA2012/050549
(87) International publication number: WO 2013/023296

(56) References cited:
- WO-A1-97/23600
- WO-A1-2011/062615
- WO-A1-2011/062615
- WO-A1-2012/027850
- WO-A2-2009/089154
- WO-A2-2009/089154
- WO-A2-2009/104074
- WO-A2-2009/104074
- KERSTIN STEINER ET AL: "Recombinant Glycans on an S-Layer Self-Assembly Protein: A New Dimension for Nanopatterned Biomaterials", SMALL, vol. 4, no. 10, 1 October 2008 (2008-10-01), pages 1728-1740, XP55151961, ISSN: 1613-6810, DOI: 10.1002/smll.200701215
- DOTAN N ET AL: "Anti-glycan antibodies as biomarkers for diagnosis and prognosis", LUPUS, BASINGSTOKE, GB, vol. 15, no. 7, 1 January 2006 (2006-01-01), pages 442-450, XP009135506, ISSN: 0961-2033
- MARIO F FELDMAN ET AL: "Engineering N-linked protein glycosylation with diverse O antigen lipopolysaccharide structures in Escherichia coli", PROCEEDINGS OF THE NATIONAL ACADEMY OF SCIENCES, NATIONAL ACADEMY OF SCIENCES, US, vol. 102, no. 8, 22 February 2005 (2005-02-22), pages 3016-3021, XP009121034, ISSN: 0027-8424, DOI: 10.1073/PNAS.0500044102 [retrieved on 2005-02-09]
- IWASHKIW ET AL.: 'Exploiting the Campylobacter jejuni protein glycosylation system for glycoengineering vaccines and diagnostic tools directed agains brucellosis' MICROBIAL CELL FACTORIES vol. 11, no. 13, 25 January 2012, pages 1 - 11, XP021118468
- CIOCCHINI ET AL.: 'A novel, rapid and simple magnetic bead-based assay for the diagnosis of human and animal brucellosis' BRUCELLOSIS INTERNATIONAL RESEARCH CONFERENCE September 2011, BUENOS AIRES, ARGENTINA, XP008173150
- FELDMAN ET AL.: 'Engineering N-Linked protein glycosylation with diverse O antigen lipopolysaccharide structures in Escherichia coli' PNAS vol. 102, no. 8, 22 February 2005, pages 3016 - 3021, XP009121034
- HUG ET AL.: 'Analogies and homologies in lipopolysaccharide and glycoprotein biosynthesis in bacteria' GLYCOBIOLOGY vol. 21, no. 2, 24 September 2010, pages 138 - 151, XP055142694
- FISHER ET AL.: 'Production of Secretory and Extracellular N-Linked Glycoproteins in Escherichia coli' APPLIED AND ENVIRONMENTAL MICROBIOLOGY vol. 77, no. 3, 03 December 2010, pages 871 - 881, XP055142701
- IWASHKIW, J.: 'Glycoengineering a novel brucellosis glycoconjugate vaccine' POSTER, GORDON RESEARCH CONFERENCE IN GLYCOBIOLOGY 2009, VENTURA, CA,

## Description

### FIELD

The present invention pertains to the field of diagnostics, particularly methods and systems for diagnostic testing of bacterial infections in organisms, including humans, using recombinant bacterial glycoproteins.

### BACKGROUND

Pathogenic bacteria have been responsible for millions of deaths of humans, cattle and other animals. Infection from bacteria has caused such diverse conditions as the bubonic plague, pneumonia, tuberculosis, anthrax, and methicillin-resistant Staphylococcus aureus (MRSA) infection which is becoming increasingly problematic in hospital environments. Thus, it is important to diagnose and treat bacterial infections to reduce the burden on the health-care system and the population as a whole.

There have been numerous methods developed that can be used to diagnose bacterial infections. Common tests include sputum, blood and urine tests which, historically, offer limited information regarding the severity of the infection. Tests can be non-specific as to the identity of the infecting bacteria and are often inaccurate. Such tests can be designed to detect the presence of the bacteria directly (e.g., by detecting a characteristic protein or nucleic acid), or indirectly, for example, by detecting antibodies that the infected individual has developed in response to the bacterial infection.

Immunoassays using lipopolysaccharide (LPS) from bacteria as an antigen, have been developed (see, for example, Lindberg, et al. "Enzyme immunoassay of the antibody response to Brucella and Yersinia enerocolitica 09 infections in humans" J. Hyg. 1982, 88(2):295 - 307; and Lindberg, et al. "Shigellosis in Vietnam: seroepidemiologic studies with use of lipopolysaccharide antigens in enzyme immunoassays" Rev. Infect. Dis. 1991, 13 Suppl 4:S231-S237). LPSs are large molecules that comprise an O antigen (or O polysaccharide), a core polysaccharide and Lipid A. LPS is the major component of Gram-negative bacteria and, consequently, recombinant or isolated LPS has been used in immunoassays for Gram-negative bacteria. However, these assays suffer from false positives as a result of the high immunogenicity of the LPS antigen (see, for example, Eoh, et al. "Expression and Validation of D-Erythrulose 1-Phosphate Dehydrogenase from Brucella abortus: A Diagnostic Reagent for Bovine Brucellosis" 2010, 22(4):524 - 530). For example, antibodies against the Lipid A core often cause false positives in immunoassays that employ LPS.

The use of recombinant bacterial glycoproteins to diagnose Gram-negative bacterial infections has been previously suggested by Castric *et al.* in U.S. Patent Publication No. U.S. 2002/0039755 and International PCT application publication number WO 1997/023600. However, this suggested use has serious disadvantages. In Castric *et al*., it is suggested that Gram-negative bacterial infections can be diagnosed by detecting antibody complexes formed between pilin-glycan conjugates produced using the oligosaccharyl transferase enzyme PilO from *Pseudomonas aeruginosa.* No actual assay was performed in Castric *et al.* Unfortunately, reliance on PilO severely limits both the type of O-antigen repeating units and the type of glycoprotein that can be detected. PilO only transfers short glycan chains to the C-terminal serine residue of one protein, specifically pilin (Castric *et al.* in U.S. Patent Publication No. U.S. 2002/0039755 and International PCT application publication number WO 1997/023600, Faridmoayer, et al. "Functional Characterization of Bacterial Oligosaccharyltransferases Involved in O-Linked Protein Glycosylation" J. Bacteriol, 2007, 189(22): 8088-8098). This limitation would severely restrict the type of bacterial infections that may be detected and the sensitivity of the assay, if the assay were even functional.

There remains a need for additional diagnostic assays for detection of bacterial infection.

WO2011/062615 discloses bioconjugate vaccines comprising N-glycosylated proteins. WO2009/104074 discloses a bioconjugate vaccine, such as an Ol-bioconjugate vaccine. Steiner et al "Recombinant Glycans on an S-Layer Self-Assembly Protein: A New Dimension for Nanopatterned Biomaterials", SMALL, (2008), vol. 4, no. 10, pages 1728 - 1740 discloses experiments investigating the integration of recombinant carbohydrates into a "biomolecular construction kit" for the design of new biomaterials, by utilizing the self-assembly system of the crystalline cell surface (S)-layer protein SgsE of Geobacillus stearothermophilus NRS 2004/3a. Dotan et al "Anti-glycan antibodies as biomarkers for diagnosis and prognosis", LUPUS, (2006), vol. 15, no. 7, pages 442 - 450 discloses the use of anti-glycan antibodies as biomarkers. WO2009/089154 discloses a prokaryotic host cell comprising eukaryotic glycosyltransferase activity, where the eukaryotic glycosyltransferase activity is eukaryotic dolichyl-linked UDP-GIcNAc transferase activity and eukaryotic mannosyltransferase activity. Feldman et al "Engineering N-linked protein glycosylation with diverse O antigen lipopolysaccharide structures in Escherichia coli", (2005) PNAS, vol. 102, no. 8, pages 3016 - 3021 discloses engineered *E. coli* cells so that two different pathways, protein N-glycosylation and lipopolysaccharide (LPS) biosynthesis, converge at the step in which Pg1B, the key enzyme of the *C*. *jejuni* N-glycosylation system, transfers O polysaccharide from a lipid carrier (undecaprenyl pyrophosphate) to an acceptor protein.

An object of the present invention is to provide a method of diagnosing bacterial infections using engineered glycoproteins. The scope of present invention is defined with reference to the attached claims.

Disclosed is a method of diagnosing bacterial infections using engineered glycoproteins in an immunoassay. The engineered bacterial glycoproteins used in the immunoassay comprise a bacterial antigen covalently attached to a protein via polysaccharyltransferase (PTase)-mediated glycosylation, wherein the bacterial antigen is selected based on the bacterial infection of interest. Antibodies in a bodily fluid of subjects infected with a bacteria will bind to the engineered glycoprotein, and the resulting binding complexes are detected or quantitated.

Disclosed is a method of detecting a bacterial infection in a subject suspected of having a bacterial infection, the method comprising: contacting the sample with a mixture of one or more glycoproteins; and detecting the presence or absence of a bacterial infection by detecting the presence or absence of a complex formed between the glycoproteins and antibodies specific therefor in the sample, wherein each of said glycoproteins comprises a bacterial antigen covalently attached to a protein via polysaccharyltransferase (PTase)-mediated glycosylation.

Optionally, the step of detecting antibody-glycoprotein binding complexes comprises incubating the sample and one or more glycoproteins with a secondary antibody that specifically binds to the anti-bacterial antibody or to the antibody-glycoprotein binding complexes.

Also disclosed is an immunoassay kit for diagnosing a bacterial infection in a subject, the kit comprising: a mixture of one or more glycoproteins, wherein each glycoconjugate comprises a bacterial antigen covalently attached to a protein via polysaccharyltransferase (PTase)-mediated glycosylation; and instructions for contacting the mixture of glycoconjugates with a sample of bodily fluid from the subject and monitoring for formation of binding complexes formed by binding of the glycoproteins by antibodies in the sample that are specific for bacterial polysaccharides.

Further disclosed is a kit for diagnosing a bacterial infection in a subject, the kit comprising: a solid medium comprising having one or more glycoproteins immobilized on a surface thereof, wherein each of said glycoproteins comprises a bacterial antigen covalently attached to a protein via polysaccharyltransferase (PTase)-mediated glycosylation; and instructions for contacting the solid medium with a sample of bodily fluid from the subject and monitoring for formation of binding complexes formed by binding of the glycoproteins by antibodies in the sample that are specific for bacterial polysaccharides.

Also disclosed is a method for diagnosing a bacterial infection in a subject comprising: contacting a sample obtained from the subject with a glycoprotein comprising a bacterial polysaccharide covalently attached to a protein; and detecting first binding complexes formed by binding of the bacterial polysaccharide in the glycoprotein to antibodies present in the sample that are specific for the bacterial polysaccharide, wherein the bacterial polysaccharide and the protein are from different bacterial species, and wherein the presence of binding complexes is indicative of a bacterial infection in the subject.

### BRIEF DESCRIPTION OF THE FIGURES

Figure 1 shows a schematic representation of the immune based assay with the Cy5 secondary antibody.
Figure 2 shows results of an immunoassay using AcrA-AO157 coated paramagnetic microbeads and a Cy5 conjugated secondary antibody. Eight patients with HUS (E1 to E8) and 4 healthy individuals (S1 to S4) are shown. All sera were used in a 1:500 dilution.
Figure 3 shows a Western blot with the sera from the HUS (E) and the healthy (S) patients. NG, non-glycosylated AcrA; G, glycosylated AcrA (AcrA-AO157). All sera were used in a 1:500 dilution.
Figure 4 shows results of an immunoassay using AcrA-AO9 coated paramagnetic microbeads and a Cy5 conjugated secondary antibody. Naive and Δ*pgm* vaccinated animals pre and post-infection with the wild type *B. abortus* 2308 strain are shown.
Figure 5 shows a Western blot with the sera of vaccinated and non-vaccinated, pre and post-infection. NG, non-glycosylated AcrA; G, glycosylated AcrA (AcrA-AO9).
Figure 6 shows results of an immunoassay using AcrA-AO9 coated paramagnetic microbeads and a Cy5 conjugated secondary antibody. Sera from four patients with a confirmed *Brucella* infection (A, B, C and D) and joint fluid from a *Brucella* knee abscess were tested.
Figures 7A-C show the AcrA-OAg glycoconjugate as the antigen for diagnosis of human brucellosis.
Figures 8A and 8B show the results of glycoconjugate magnetic bead assay analyses of serum samples obtained from healthy individuals and patients from different clinical groups.
Figures 9A and 9B show the results of a receiver-operating characteristic (ROC) analysis carried out using a glycoconjugate magnetic bead assay.
Figure 10 shows the results of an assay of serum samples from animals experimentally infected with *B. abortus* 2308; the results are shown as a percentage activity as described in Example 5.
Figure 11 shows the Western blot analysis results of the bovine serum assay as described in Example 5.
Figure 12 shows the results of analysis of bovine serum and whole blood samples obtained 6 months post-infection.
Figure 13 shows the assay results from animals (bovine) vaccinated with the smooth strain S19 Serum samples.
Figure 14 shows the assay results from animals vaccinated with S19 and challenged with *B. abortus* 2308; the assay was perfomed using milk samples.
**Figure 15** **shows the assay results from animals vaccinated with a rough strain** (Δ*pgm*); the assay was perfomed using serum samples.

### DETAILED DESCRIPTION

### Definitions

Unless defined otherwise, all technical and scientific terms used herein have the same meaning as commonly understood by one of ordinary skill in the art to which this invention belongs.

As used in the specification and claims, the singular forms "a", "an" and "the" include plural references unless the context clearly dictates otherwise.

The term "comprising" as used herein will be understood to mean that the list following is non-exhaustive and may or may not include any other additional suitable items, for example one or more further feature(s), component(s) and/or ingredient(s) as appropriate.

As used herein, the term "immunoassay" refers to biochemical test used to qualitatively or quantitatively detect the presence of a substance in a sample.

As used herein, the term "target antibody" refers to an antibody specific for a bacterial antigen from a bacteria suspected of infecting a subject. It is the presence or absence of the target antibody that is used as an indicator of bacterial infection in the subject.

As used herein, the term "bodily fluid" refers to a naturally occurring and/or secreted and/or excreted and/or discharged fluid and/or wash fluid from the surface or inside the bodies of a human or an animal and includes, but is not limited to: serum, plasma, blood, synovial fluid, pharyngeal, nasal/nasal pharyngeal and sinus secretions, saliva, sputum, urine, mucous, feces, chyme, vomit, gastric juices, pancreatic juices, semen/sperm, cerebral spinal fluid, products of lactation or menstruation, egg yolk, amniotic fluid, aqueous humour, vitreous humour, cervical secretions, vaginal fluid/secretions, bone marrow aspirates, pleural fluid, sweat, pus, tears, lymph and bronchial or lung lavage.

As used herein, a "glycoprotein" is a molecule comprising protein with covalently bound glycans.

As used herein, a "glycan" comprises any sugar that can be transferred (e.g, covalently attached) to a protein. A glycan can be a monosaccharide, oligosaccharide or polysaccharide. As described above, an oligosaccharide is a glycan having 2 to 10 monosaccharides. A polysaccharide is a glycan having greater than 10 monosaccharides. Polysaccharides can be selected from the group comprising O-antigens, capsules, and exopolysaccharides. Of course, one of skill in the art will appreciate that other types of polysaccharides may also be used.

When the glycoprotein is used as a component of the assay system described herein, at least a portion of the attached glycans are antigenic. This is described in more detail below.

Glycans useful herein include, but are not limited to, glycans from *Brucella abortus, Yersinia pseudotuberculosis* O9, *C*. *jejuni, N. meningitidis, P. aeruginosa, S. enterica* LT2, and *E*. *coli.* In one embodiment, the monosaccharide at the reducing end of the glycan is a hexose or an N-acetyl derivative of a hexose. In one aspect, the hexose can be galactose. In one aspect, the N-acetyl derivative of hexose can be selected from the group comprising N-acetylglucosamine (GIcNAc), 2-Acetamido-2,6-dideoxyhexose (FucNAc), and DATDH (2,4- diacetamido-2,4-,6-trideoxyhexose).

Disclosed is an immunological method for exploiting recombinant bacterial glycoproteins to detect antibodies directed against bacterial polysaccharides present in fluids of infected organisms. The method is useful for identifying or quantifying bacterial infection in a subject, such as a human or livestock.

The surface of bacteria includes polysaccharides, many of which are antigenic and will trigger an immune response upon infecting a host organism. That is, the infection will trigger the production of host antibodies specific for bacterial surface polysaccharides and glycoproteins. The present assay makes use of recombinant bacterial glycoproteins in an assay to detect such host antibodies.

While other methods for assaying and diagnosing bacterial infections have been described, none to date exploit the use of bacterial glycoproteins, particularly those generated by the activity of polysaccharyltransferases (PTases). The assay described provides a method of detecting bacterial antibodies in infected subjects.

The examples provided below demonstrate the production of recombinant glycoproteins and their successful use in the presently described assay in detecting *Brucella* and *E. coli* infections in humans and animals. This is indicative of the successful use of the assay for detecting any bacterial infection in a human or animal. For example, the present assay can be used to detect infection by any clinically important bacteria, which may be, for example, *Salmonella, E. coli, Brucella, Shigella, Vibrio cholera, Neisseria meningitidis, Klebsiella, Citrobacter, Hafnia, Proteus, Haemohilus influenzae, Streptococcus pneumoniae, Staphylococcus,* Group B streptococci, *Yersinia pseudotuberculosis, Campylobacter jejuni, Acinetobacter baumannii* and *Pseudomonas aeruginosa.* These bacteria have antigenic polysaccharides covering all or a portion of their surface.

### Glycoproteins

The glycoproteins used in the immunological method described herein contain a glycosylated protein made by via PTase-mediated glycosylation. In particular, the present inventors have taken advantage of the promiscuity or flexibility of PTases to manufacture glycoproteins comprising protein glycosylated with bacterial antigens.

Protein glycosylation is a common posttranslational modification in bacteria, including both gram-negative and gram-positive bacteria. While the precise functions of the glycans attached to bacterial proteins have not been determined, glycoproteins may play roles in adhesion, stabilization of the proteins against proteolysis, and evasion of the host immune response (Faridmoayer et al., "Functional characterization of bacterial Oligosaccharyltransferases involved in O-linked protein glycosylation." J. Bacteriology 2007;189(22):8088-8098).

Glycans are generally attached to serine or threonine (O-glycosylation) or asparagine (N-glycosylation) residues. Two different mechanisms for protein glycosylation can be distinguished by the mode in which the glycans are transferred to proteins. The first mechanism involves the transfer of carbohydrates directly from nucleotide-activated sugars to acceptor proteins. Examples of pathways using this mechanism are protein O-glycosylation in the Golgi apparatus in eukaryotic cells, and flagellin O-glycosylation in several bacterial species. In the second mechanism, an oligosaccharide is preassembled onto a lipid carrier before being transferred to protein acceptors by an oligosaccharyltransferase (OTase)or polysaccharyltransferase (PTase). This mechanism has been described for N-glycosylation in the endoplasmic reticulum of eukaryotic cells and in the general N-glycosylation system of *Campylobacter jejuni.*

Bacterial OTases and PTases constitute a family of enzymes that serve in linking a wide variety of glycans to proteins in both prokaryotes and eukaryotes (Feldman et al. "Engineering N-linked protein glycosylation with diverse O antigen lipopolysaccharide structures in Escherichia coli." Proc. Nat. Acad. Sci. 2005;102(8):3016-21; Wacker et al., "Substrate specificity of bacterial oligosaccharyltransferase suggests a common transfer mechanism for the bacterial and eukaryotic systems." Proc. Nat. Acad. Sci. 2006;103(18)7088-93; Faridmoayer et al., "Extreme substrate promiscuity of the Neisseria Oligosaccharyl Transferase Involved in Protein O-Glycosylation." J. Biol. Chem. 2008;283 (50):34596-34604; Iwashkiw poster, "Glycoengineering a novel brucellosis glycoconjugate vaccine." Gordon Research Conference in Glycobiology, Ventura, CA, 2009).

Three bacterial oligosaccharyl and polysaccharyl transferases have been characterized to date: namely, *Campylobacter* PglB, *Neisseria* PglL; and *Pseudomonas* PilO. PglB attaches sugars to asparagine residues and belongs to a different family of PTases, similar to the eukaryotic transferases, than PglL and PilO. Both PglL and PilO attach sugars to serine residues and include a homologous domain. However, PglL and PilO differ significantly, for example in the glycans they are capable of transferring. PilO only transfers short glycan chains (i.e., oligosaccharides) to the C-terminal serine residue of one known protein (pilin) (Faridmoayer (2007), *supra*). As a result, this enzyme is referred to herein as an OTase. In contrast, PglL can transfer virtually any sugar including polysaccharides to internal serine residues in different proteins. Both PglB and PglL are referred to herein as PTases since they are capable of transferring polysaccharides to proteins.

In the immunoassay defined herein, engineered glycoproteins have been designed to be reactive with antibodies present in samples obtained from individuals having a bacterial infection. Polysaccharides are capable of acting as efficient binding partners with antibodies, whereas oligosaccharides are not efficient binding partners, which makes them unsuitable for use in sensitive immunoassays. Accordingly, the OTase PilO is not suitable for use in the manufacture of glycoproteins used in the present immunoassay. Rather PTases, such as PglB and PglL are effective in generating glycoproteins containing different polysaccharides, which are successfully used in the present immunoassay. Exemplary embodiments of the assay are described below.

The engineered glycoproteins useful in the present immunoassay can be prepared using PTases according to known methods (see, for example, PCT Publication WO2011/062615; US Patent Publication 2009/0735773; US Patent Publication 2009/0074798; US Patent Publication 2005/0192962; US Patent Publication 1999/0337393; PCT Publication WO/1997/023600, Wacker, *supra;* Faridmoayer, *supra.*

Generally, engineered glycoproteins are prepared by incubating a PTase with a selected acceptor protein and a lipid-linked glycan. The method can comprise introducing into a prokaryotic organism, in any particular order, at least a DNA comprising a gene that produces a PTase, and DNA comprising a gene that produces a protein to be glycosylated. The PTase facilitates the covalent attachment of the glycan(s) to the protein to produce the antigenic glycoprotein. When such glycoproteins are to be employed in an immunoassay as described herein the glycan comprises a polysaccharide, or combination thereof, identified as being immunogenic and/or suspected of being immunogenic.

The PTase makes use of a lipid carrier to transfer the glycan to the acceptor protein. In certain embodiments, the lipid carrier is a polyprenol-pyrophosphate including, but not limited to, undecaprenol-pyrophosphate, dolichol-pyrophosphate, and synthetic equivalents thereof. In other embodiments, the method further comprises introducing into the prokaryotic organism DNA comprising genes required for the assembly of a glycan onto a lipid carrier.

The use of PTases is particularly advantageous since they can be used to prepare glycosylated proteins without introducing limitations as to the type of glycan that can be added to proteins, the length of the glycan transferred, the type of sugar located at the reducing end of the glycan, the position of the glycan on the protein or the type of organisms that can be used.

The acceptor protein in the engineered glycoprotein can be any protein that functions as a substrate for PTase and, preferably, exhibits low immunogenicity in comparison to the polysaccharide. The low immunogenicity allows the polysaccharide component of the engineered glycoprotein to function as the antigenic component and minimizes false positives that can be caused by non-specific antibody binding during the immunoassay.

In a specific embodiment, the acceptor protein is the *N*-glycoprotein AcrA protein of *Campylobacter jejuni.* For the glycoprotein can comprise the AcrA of *C. jejuni* coupled to an O-antigen of *Yersinia pseudotuberculosis* O9 (AcrA-AO9), or a polysaccharide antigen of *E. coli* (e.g., AcrA-AO157).

### Immunoassay

In accordance with one aspect, there is provided an immunological method, or an immunoassay system, for detecting in a sample of bodily fluid antibodies specific for a bacterial antigen. The presence or quantity of such antibodies can be useful in diagnosing bacterial infection in the subject from which the bodily fluid sample was obtained. The immunoassay described herein employs the engineered glycoprotein described above to form a binding complex with antibodies present in a bodily fluid sample that are specific for a bacterial antigen. The glycan component of the engineered glycoprotein used in the immunoassay is selected based on the specific bacterial infection to be assayed.

Immunoassays for detecting a target antibody of interest from samples may be either competitive or noncompetitive. In either case, the presence of a complex formed between the engineered glycoprotein described above and antibodies specific therefor can be detected by any of the known methods common in the art, such as, for example, fluorescent antibody spectroscopy or colorimetry.

As is well known in the field, noncompetitive immunoassays are assays in which the amount of captured target antibody is directly measured. In one preferred assay, for example, the engineered glycoprotein specific for the target antibody can be bound directly (immobilized) to a solid substrate or solid medium where the antibody will be immobilized upon forming a binding complex with the engineered glycoprotein. The target antibody/engineered glycoprotein complex thus immobilized is then bound by a labeling agent, such as a second or third antibody bearing a label.

In competitive assays, the amount of target antibody in a sample is measured indirectly by measuring the amount of an added (exogenous) target antibody displaced (or competed away) from a engineered glycoprotein specific for the target antibody by the target antibody present in the sample. In a typical example of such an assay, the engineered glycoprotein is immobilized and the exogenous target antibody is labeled. Since the amount of the exogenous target antibody bound to the glycoprotein is inversely proportional to the concentration of the target antibody present in the sample, the target antibody level in the sample can thus be determined based on the amount of exogenous target antibody bound to the glycoprotein and, thus, immobilized.

As would be readily appreciated by a worker skilled in the art, other assay formats can be used that fall within the present immunoassay provided that they can take advantage of the formation of binding complexes between the present engineered glycoprotein and a target antibody to identify or quantify bacterial infection.

The immunoassays described herein typically utilize a labeling agent to specifically bind to and label the binding complex formed by the target antibody and the engineered glycoprotein. The labeling agent may itself be one of the moieties comprising the antibody/glycoprotein complex, or may be a third moiety, such as another antibody, that specifically binds to the antibody/glycoprotein complex. A label may be detectable by, for example, spectroscopic, photochemical, biochemical, immunochemical, electrical, optical or chemical means. Examples include, but are not limited to, fluorescent dyes (e.g., fluorescein isothiocyanate, Texas red, rhodamine, and the like), radiolabels (e.g., ³H, ¹²⁵I, ³⁵S, ¹⁴C, or ³²P), enzymes (e.g., horse radish peroxidase, alkaline phosphatase, and others commonly used in an ELISA), luminescent dyes, and colorimetric labels such as colloidal gold or colored glass or plastic (e.g., polystyrene, polypropylene, latex, etc.) beads.

In some cases, the labeling agent is a secondary antibody bearing a detectable label. Alternatively, the secondary antibody may lack a label, but it may, in turn, be bound by a labeled third antibody specific to antibodies of the species to which the secondary antibody corresponds. Alternatively, the secondary antibody can be modified with a detectable moiety, such as biotin, to which a third labeled molecule can specifically bind, such as enzyme-labeled streptavidin.

Other proteins capable of specifically binding immunoglobulin constant regions, such as protein A or protein G, can also be used as the label agents. These proteins are normal constituents of the cell walls of streptococcal bacteria. They exhibit a strong non-immunogenic reactivity with immunoglobulin constant regions from a variety of species (see, generally, Kronval, et al. J. Immunol., 111: 1401-1406 (1973); and Akerstrom, et al., J. Immunol., 135: 2589-2542 (1985)).

As noted above, the immunoassay optionally includes the use of immobilized engineered glycoprotein. In this embodiment, the engineered glycoprotein is bound directly to a solid support, which may be, for example, the surface of microtitre plates, polymer beads, agarose beads, paramagnetic (or magnetic) beads, or membranes. The engineered glycoproteins can be immobilized on the surface of the solid support by various means. In one embodiment, the glycoconjugates are covalently bound to the solid support. For example, the surface of the solid support can be surface modified to include any typical modifying group, such as a carboxyl group, that reacts with the amino groups (-NH₂) of the protein in the engineered glycoprotein. In a specific, non-limiting example, the engineered glycoproteins can be covalently bound to a solid surface that is surface-modified with carboxyl group, by a one step reaction is performed using 1-ethyl-3-(3-dimethyl-aminopropyl) carbodiimide hydrochloride as a catalyzer.

In accordance with another aspect there is a provided an immunoassay kit comprising engineered glycoproteins as described above and reagents for the detection of binding complexes formed with the engineered glycoproteins and antibodies specific for bacterial antigens corresponding with the antigenic components of engineered glycoproteins. The immunoassay kit can additionally include instructions for use and/or a container or other means for collecting a sample of bodily fluid from a subject suspected of having a bacterial infection.

To gain a better understanding of the invention described herein, the following examples are set forth. It should be understood that these examples are for illustrative purposes only. Therefore, they should not limit the scope of this invention in any way.

### Examples

### Example 1 - Immunoassay

The assay described herein is based on antigen-antibody interaction, as are all immunoassays. In the embodiment of this example, engineered glycoproteins are covalently linked to paramagnetic surface-modified microbeads.

In use, a typical assay in accordance with the present embodiment is summarized as follows:
1) the modified microparticles are incubated with serum to be analyzed;
2) the serum-incubated microparticles are then washed and incubated with secondary antibodies, which are conjugated with a reporter molecule, such as peroxidase or Cy5; and
3) the serum-incubated microparticles from 2) are washed again and developed either by, for example, fluorescence (Cy5) or electrochemically (peroxidase).

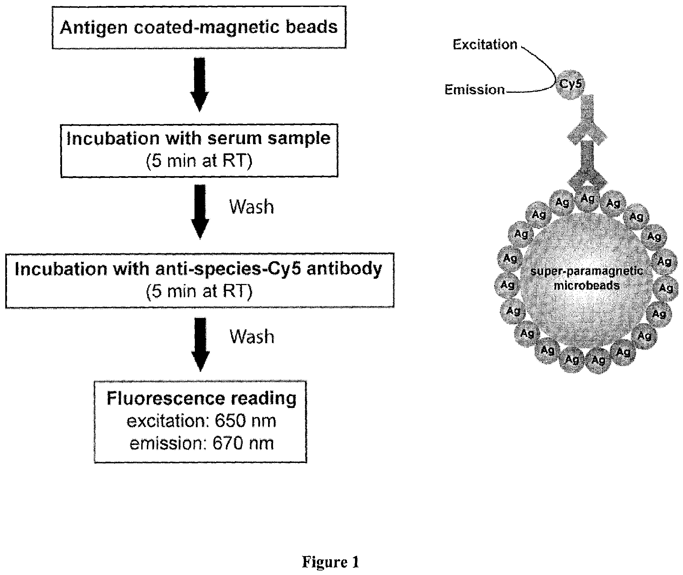

A schematic representation of this exemplary assay is shown in Figure 1.

Briefly, a dilution of the serum to be assayed (1:100 to 1:500 depending on the sample and the antigen used) is incubated for 5 min with the nanobeads coated with a engineered glycoprotein. After this incubation, the microbeads are pelleted with a magnetic field; washed; and resuspended in the presence of the secondary antibody and incubated for 5 min. The beads are pelleted again with a magnetic field, washed three times with buffer PBS and either measured directly in a spectrofluorometer if a Cy5 secondary antibody is used or incubated with oxygen peroxide and measured electrochemically if a secondary conjugated to peroxidase is used.

### Example 2 - Escherichia coli 0157 assay

The enterohemorragic *Escherichia coli* 0157 is a common foodborne pathogen associated with the hemolytic uremic syndrome (HUS), with a high mortality rate, especially among children under 5 years old. The disease is preceded by an episode of diarrhea, usually with the presence of blood. To date there is no rapid and robust test to diagnose this important human pathogen in the acute phase of the diarrhea.

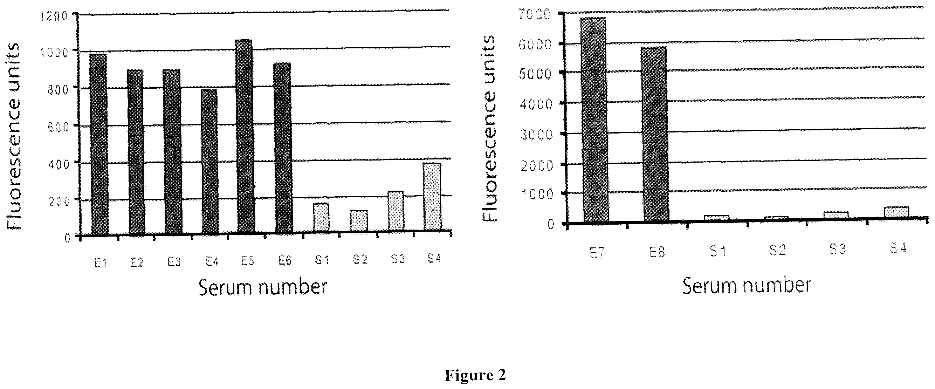

To assay for *E. coli* 0157, the AcrA-AO157 engineered glycoprotein antigen was coupled to magnetic microbeads. The AcrA-A0157 glycoprotein includes the AcrA protein of C. *jejuni* coupled to an *E. coli* glycan using a PTase. This immune test was used to detect an *E. coli* 0157 infection as early as 2 days post-bloody diarrhea. As shown in Figure 2, sera of patients that developed the hemolytic uremic syndrome as a consequence of an *E. coli* 0157 infection tested positive in the present assay.

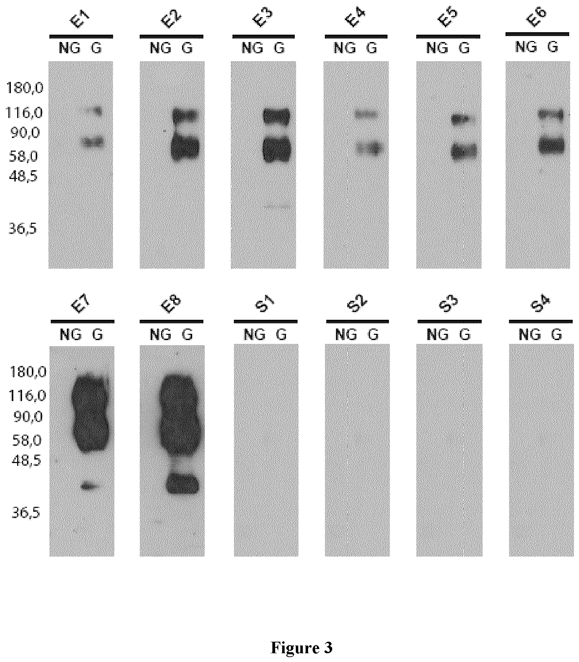

Figure 3 shows that the same sera reacted with the AcrA-AO157 engineered glycoprotein but not with non-glycosylated AcrA, demonstrating that the reaction is specific towards the glycosidic part of the antigen.

### Example 3 - Brucella spp. assay

*Brucella* spp. is the etiological agent of Brucellosis, a wide spread zoonosis that affects a broad range of mammals, including humans. *Brucella abortus* infects bovines and causes abortion in pregnant animals inflicting significant economic losses in developing countries, in some of which it is still endemic. Although there are many diagnostic assays for cattle and humans known, it is often necessary to run several of them in parallel to have a confirmed diagnosis. This can be a complicated and expensive exercise.

A novel engineered glycoprotein, AcrA-AO9, was constructed, which has the AcrA protein of *C. jejuni* coupled to the O-antigen of *Yersinia pseudotuberculosis* O9 (which has the same structure than the *Brucella* O-antigen). Synthesis of this engineered glycoprotein is described previously (Feldman *et al*., supra). This antigen was coupled to paramagnetic microbeads and tested with sera of vaccinated and infected cows, as well as with sera of humans with acute or chronic Brucellosis.

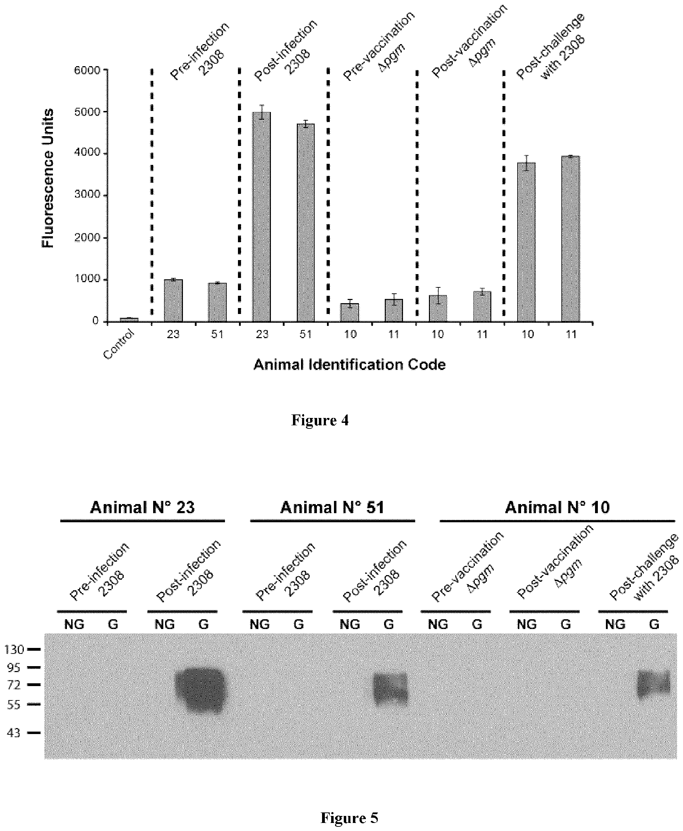

Figure 4 shows the results of assays performed with sera from vaccinated and non-vaccinated animals before and after infection with the wild type *B. abortus* 2308 *Brucella* strain. **The Δpgm vaccine strain is deficient in the assembly of the O-antigen and, due to this, no** reactivity against the glycoprotein should be observed. As shown in Figure 4, post-infection of both non-vaccinated and vaccinated animals with the wild type strain were detected with the **assay as presently described. As expected, animals vaccinated with Δpgm did not demonstrate** any measurable detection levels.

Figure 5 is a western blot performed with these sera demonstrating that the reactivity is against the glycosidic part of the antigen.

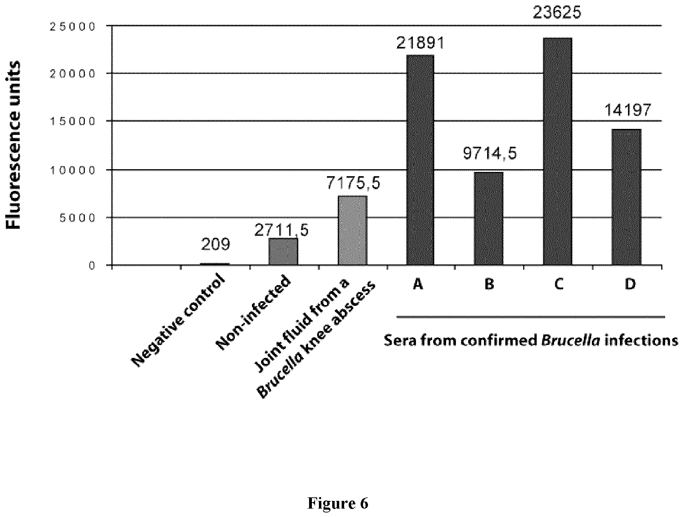

To demonstrate that the immunoassay of the present example is suitable to diagnose human Brucellosis, sera from 4 confirmed human infections and one knee synovial fluid product of a *Brucella abortus* abscess were analyzed. As shown in Figure 6, all samples tested were positive for detecting Brucellosis in human infections.

### Example 4 - Diagnosis of Human Brucellosis

Brucellosis commonly infects humans and can cause severe symptoms such as sweating, fatigue, anorexia, weight loss, headache, arthralgia and generalized aching. In severe cases, Brucellosis can cause abscesses, endocarditis and neurobrucellosis, sometimes leading to death. Early detection in humans would be very helpful in decreasing the severity and prevalence of the disease in human populations.

An indirect immunoassay was developed to detect antibodies against "smooth" *Brucella spp.* using as an antigen a recombinant O-polysaccharide-protein conjugate (AcrA-OAg) coupled to magnetic beads. AcrA-OAg was produced in *Escherichia coli* by co-expressing PglB, a *Campylobacter jejuni* oligosaccharyltransferase, the gene cluster encoding the enzymes required for the synthesis of *Brucella abortus* O-polysaccharide and the protein acceptor AcrA. Introduction of PglB in *E. coli* results in the transfer of *B. abortus* O-polysaccharide from its carrier to AcrA. The AcrA-OAg glycoprotein, expressed as an histidine-tag fusion protein, can then be purified from *E. coli* fermentations without the need for culturing the pathogenic bacteria. AcrA-OAg was purified in one-step by affinity chromatography and immobilized on carboxy-modified magnetic beads. Functionalized micro-beads were incubated with the samples and the immune complexes were detected using anti-human or anti-bovine IgM/G Cy5 conjugate antibodies.

### Glycoconjugate detection compared to standard serological tests for Brucella

The use of the AcrA-OAg glycoconjugate as an antigen for the diagnosis of human brucellosis was evaluated. A glycoconjugate magnetic bead-based immunoassay was carried out for the detection of antibodies against the *Brucella abortus* O-antigen. In brief, magnetic beads coated with the AcrA-OAg glycoconjugate were incubated with the indicated human serum samples (dilution 1/100). Bound antibodies were detected using goat anti-human IgG Cy5-conjugated antibodies. The results are expressed as percentage of reactivity of the positive control serum.

Standard serological tests for *Brucella* were also carried out on culture-positive patients. As shown in Table I, the results of the magnetic bead assay are compared with standard laboratory tests such as SAT (serum agglutination test), TAT (tube agglutinin test), TAT-2ME (2-mercapto-ethanol tube agglutination test) and CFT (complement fixation test) are shown as titers. For the RBT (Rose-Bengal test) and BPAT (buffered antigen plate agglutination test), the results are indicated as positive (POS) or negative (NEG). The cutoff value for cELISA (competitive enzyme-linked immunosorbent assay) is 28%. As is evident from the table, the results of standard or known diagnostic laboratory assays for *Brucella sp.* varies widely, and can give false negative results in some instances.

The results for the magnetic bead assay are expressed as percentage of reactivity of the control positive serum. The notation 'ND' means that the result was not determined. The cutoff for the magnetic bead based assays is 13-14%. Above this percent, the result is considered positive.

**Table 1**

| **Case** | **Result of serological tests** | | | | | | | ***Brucella sp.* isolated** | **Magnetic bead assay (%)** |
|---|---|---|---|---|---|---|---|---|---|
| | **RBT** | **BPAT** | **SAT** | **TAT** | **TAT- 2ME** | **cELISA** | **CFT** | | |
| 1 | POS | POS | >200 | 400 | 200 | 76 | 640 | *B. suis bv 1* | 100.8 |
| 2 | NEG | NEG | NEG | 25 | NEG | 38 | ND | *B. suis bv 1* | 22.1 |
| 3 | POS | POS | >200 | 400 | 100 | 63 | 320 | *B. suis bv 1* | 64.8 |
| 4 | POS | POS | >200 | 800 | 200 | 77 | 160 | *B. abortus bv 1* | 66.5 |
| 5 | POS | POS | >200 | 200 | 25 | 32 | 5 | *B. melitensis bv 1* | 24.1 |
| 6 | POS | POS | >200 | 400 | 100 | 64 | 320 | *B. abortus bv 1* | 59.8 |
| 7 | POS | POS | >200 | 400 | 200 | 64 | 160 | *B. suis bv 1* | 78.5 |
| 8 | POS | POS | >200 | 1600 | 400 | 87 | 40 | *B. abortus* | 96.7 |
| 9 | POS | POS | 25 | 25 | NEG | 67 | 5 | *B. abortus bv 1* | 59.2 |
| 10 | POS | POS | >200 | 400 | 100 | 65 | 10 | *B. abortus bv 2* | 47.3 |
| 11 | POS | POS | 100 | 25 | 50 | ND | ND | *B. suis bv 1* | 148.0 |
| 12 | POS | POS | 100 | 50 | NEG | 75 | 10 | *B. abortus bv 1* | 50.5 |
| 13 | NEG | POS | 25 | 25 | NEG | 43 | 10 | *B. abortus bv 1* | 29.3 |
| 14 | POS | POS | 50 | 100 | NEG | 67 | 80 | *B. suis bv 1* | 78.6 |
| 15 | NEG | POS | 25 | 25 | NEG | 44 | 5 | *B. abortus bv 1* | 24.7 |
| 16 | POS | ND | 50 | 50 | 25 | ND | ND | *B. melitensis* | 67.9 |
| 17 | POS | ND | 1600 | 1600 | 50 | ND | ND | *B. melitensis* | 76.7 |
| 18 | POS | ND | 800 | 400 | 100 | ND | ND | *B. melitensis* | 64.1 |
| 19 | POS | ND | 1600 | 1600 | 100 | ND | ND | *B. melitensis* | 37.9 |
| 20 | POS | ND | 6400 | 6400 | 400 | ND | ND | *B. melitensis* | 47.2 |
| 21 | POS | ND | 800 | 800 | 50 | ND | ND | *B. melitensis* | 22.7 |
| 22 | POS | ND | 50 | 50 | 50 | ND | ND | *B. melitensis* | 58.7 |
| 23 | POS | ND | 100 | 100 | NEG | ND | ND | *B. melitensis* | 14.2 |
| 24 | POS | ND | 3200 | 3200 | NEG | ND | ND | *B. melitensis* | 73.0 |
| 25 | POS | ND | 6400 | 6400 | 50 | ND | ND | *B. melitensis* | 79.9 |

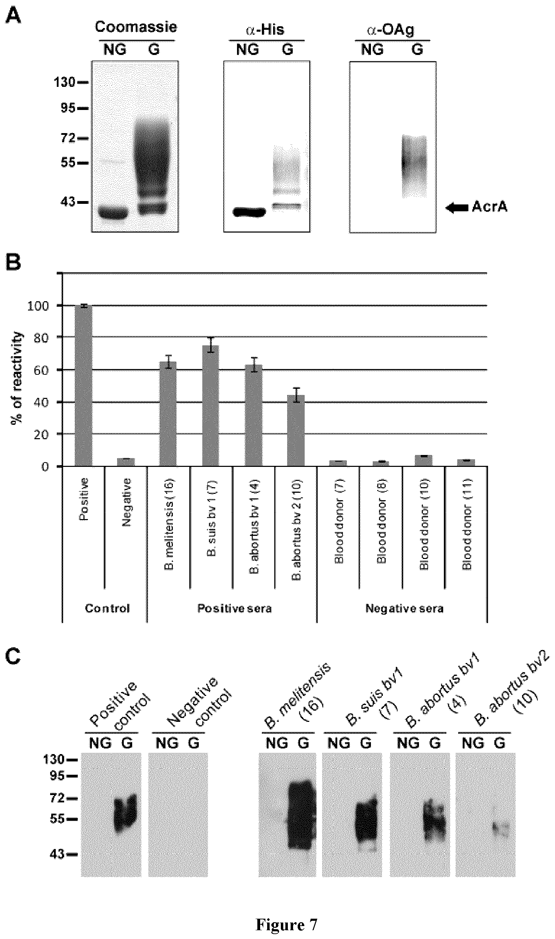

Figure 7A shows the 10 % SDS-PAGE analysis of non glycosylated (NG) and **glycosylated AcrA (G) by Coomassie brilliant blue and immunoblot using α-Histidine tag and α-***Brucella* O-antigen monoclonal antibodies.

The bar graph data shown in Figure 7B represents the mean and standard deviation for two separate determinations. A western blot analysis was performed on the same human serum samples, the results of which are shown in Figure 7C. Human sera was used for the positive and negative controls in these immunoassays.

### Detection of Brucella in Human Sera Samples

Samples of human serum were tested to determine the presence of *Brucella.* Serum samples were obtained from 413 individuals, including healthy individuals and patients from each of the following different clinical groups:
(i) Culture-positive brucellosis patients: A total of 25 sera were obtained from patients with positive blood cultures for *Brucella abortus, Brucella melitensis* and *Brucella suis* (see Table I).
(ii) Culture-negative, serologically-positive patients with clinical diagnosis of brucellosis: A total of 52 sera were obtained from patients with clinical symptoms compatible with brucellosis and with positive RBT, BPAT, SAT (Huddleson), TAT (Wright), TAT-2ME (2-mercaptoethanol), CELISA and CFT results at any titer .
(iii) Blood donors: A total of 240 sera were obtained from healthy individuals with no clinical or epidemiological evidence of brucellosis (18 to 65 years old) and with negative BPAT and SAT (Huddleson) results.
(iv) Patients with a febrile syndrome: A total of 34 serum samples were obtained from patients who initially had a clinical suspicion of brucellosis but had a different final diagnosis. These samples were negative by all the serological diagnostic tests (RBT, BPAT, SAT, TAT, TAT-2ME, cELISA and CFT).
(v) Healthy individuals occupationally-exposed to the pathogen: A total of 30 sera were obtained from research and clinical laboratory workers, and cattle ranchers.
(vi) Patients with other bacterial infections or with autoimmune pathologies: A total of 32 sera were obtained from patients infected with *Mycobacterium tuberculosis, Klebsiella pneumoniae, Acinetobacter baumannii, Pseudomonas aeruginosa, Streptococcus pneumoniae* and *Staphylococcus aureus,* and with autoimmune pathologies such as rheumatoid arthritis, systemic lupus erythematosus and autoinmune vasculitis.

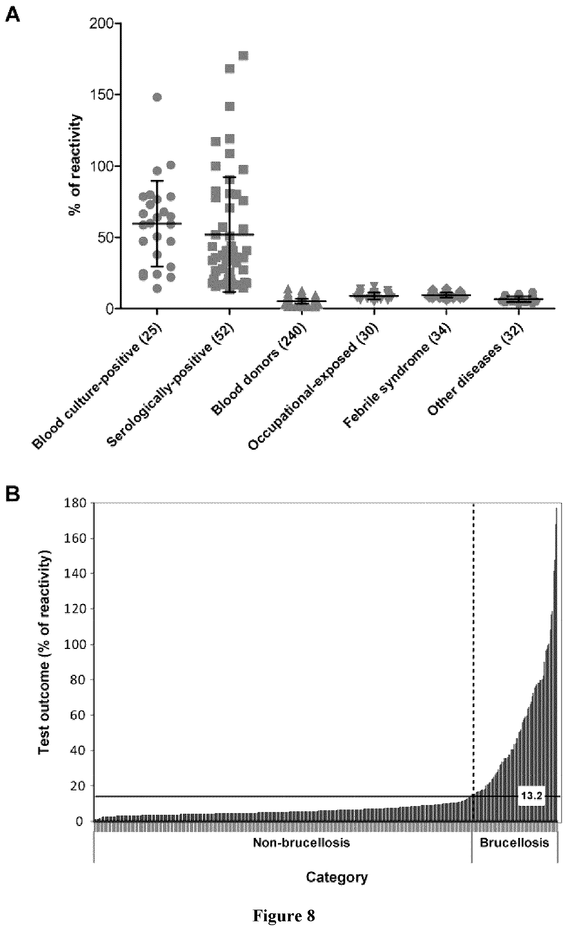

Glycoconjugate magnetic bead assay analyses of serum samples were obtained from healthy individuals and patients from different clinical groups. The results of these assays are shown in Figures 8A and 8B. Figure 8A shows a dot plot of the glycoconjugate magnetic bead assay results. Serum samples from blood culture-positive patients (25 sera), culture-negative, serologically-positive patients with clinical diagnosis of brucellosis (52 sera), blood donors (240 sera), occupational-exposed to the pathogen healthy individuals (30 sera), patients with a febrile syndrome (34 sera) and patients with other diseases (32 sera) were tested. The mean and standard deviation for each group are indicated.

Figure 8B shows a distribution of reactivity values based on the non-brucellosis or brucellosis category of the tested individual. Brucellosis category group samples were obtained from blood culture-positive as well as culture-negative, serologically-positive patients with clinical diagnosis of brucellosis. Individuals in the non-brucellosis category group include samples from blood donors, healthy individuals with occupational-exposure to the pathogen, patients with a febrile syndrome, and individuals with other diseases. For each category, reactivity values were arranged in increasing order. The horizontal line indicates the cutoff value (13.2 %) or threshold calculated by a receiver-operating characteristic (ROC) analysis. As is evident from Figure 8, a test outcome above the shown threshold of 13.2% in this assay is indicative of brucellosis.

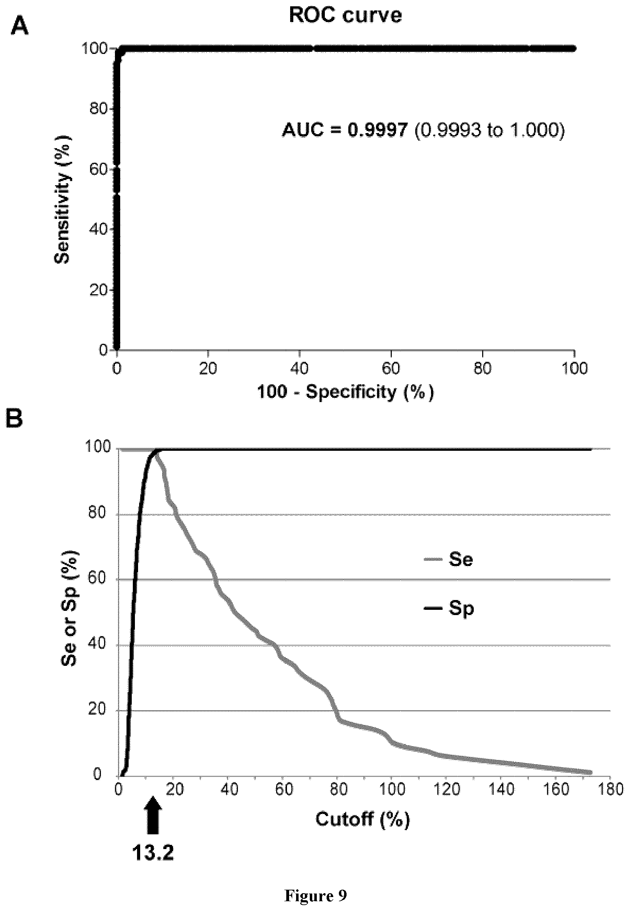

A further receiver-operating characteristic (ROC) analysis was carried out using a glycoconjugate magnetic bead assay. The results of this assay are shown in Figures 9A and 9B. As shown in the ROC plot in Figure 9A, the area under the ROC curve (AUC) is a global summary statistic of diagnostic accuracy. Values in parentheses indicate the 95% confidential interval. Figure 9B graphically depicts a plot of the diagnostic sensitivity (Se) and specificity (Sp) of the assay as a function of the cutoff value. The arrow indicates the cutoff for which the maximal sum of sensitivity (100 %) and specificity (98.81 %) values are achieved.

The sensitivity, specificity, and likelihood ratios of the test were calculated for different cutoff values. The results of these calculations are shown in Table 2. The values in parentheses as a percentage sensitivity and percentage specificity indicate the 95% confidential interval. The reported 'LR' is the likelihood ratio. Specifically, the Positive LR was calculated as sensitivity/(1.0-specificity) and the Negative LR as (1.0-sensitivity)/specificity.

**Table 2**

| **Cutoff (%)** | **Sensitivity (%)** | **Specificity (%)** | **Positive LR** | **Negative LR** |
|---|---|---|---|---|
| > 13.20 | **100.0** (95.32 - 100.0) | 98.81 (96.98- 99.67) | 84.00 | 0.0000 |
| > 13.45 | 98.70 (92.98 - 99.97) | 98.81 (96.98 - 99.67) | 82.91 | 0.0132 |
| > 13.80 | 98.70 (92.98 - 99.97) | 99.11 (97.41 - 99.82) | 110.55 | 0.0131 |
| > 14.08 | 98.70 (92.98 - 99.97) | 99.40 (97.87 - 99.93) | 165.82 | 0.0131 |
| > 14.16 | 98.70 (92.98 - 99.97) | 99.70 (98.35 - 99.99) | 331.64 | 0.0130 |
| +> 14.48 | 97.40 (90.93 - 99.68) | 99.70 (98.35 - 99.99) | 327.27 | 0.0261 |
| > 15.20 | 96.10 (89.03 - 99.19) | 99.70 (98.35 - 99.99) | 322.91 | 0.0391 |
| > 16.15 | 94.81 (87.23 - 98.57) | **100.0** (98.91 - 100.0) | - | 0.0519 |

The relationship between the test outcome and the presence or absence of disease is shown in Table 3. The 'positive' category includes culture-positive brucellosis patients (25 sera) and culture-negative, serological-positive patients with clinical diagnosis of brucellosis (52 sera). The 'negative' category includes blood donors (240 sera), patients with a febrile syndrome (34 serum samples), healthy individuals occupational-exposed to the pathogen (30 sera) and patients with other bacterial infections or with autoimmune pathologies (32 sera). The following acronyms are used in the table: TP, true positive; TN, true negative; FN, false negative; FP, false positive; Se, sensitivity; Sp, specificity; PPV, positive predictive value (TP/TP + FP) and NPV (TN/TN + FN), negative predictive value. A cutoff value of 13.2 % was taken as indicative of brucella.

**Table 3**

| | **Condition** | | |
|---|---|---|---|
| **Test outcome** | Positive | Negative | |
| Positive | 77 (TP) | 4 (FP) | **PPV:** 95.06% |
| Negative | 0 (FN) | 332 (TN) | **NPV:** 100% |
| | **Se:** 100% | **Sp:** 98.81% | |

### Example 5 - Diagnosis of bovine brucellosis

Serum, whole blood and milk samples were obtained from a vaccination/infection trial. Samples were collected from the following animal groups:
(i) Non-infected non-vaccinated;
(ii) Experimentally infected; and
(iii) Experimentally vaccinated/challenged animals.

Three different samples were obtained for each animal in the trial: a pre-infection sample, a sample obtained 21 days after infection and finally a sample obtained 6 months post-infection. A similar assay was performed using the AcrA-OAg glycoconjugate as described in Example 4.

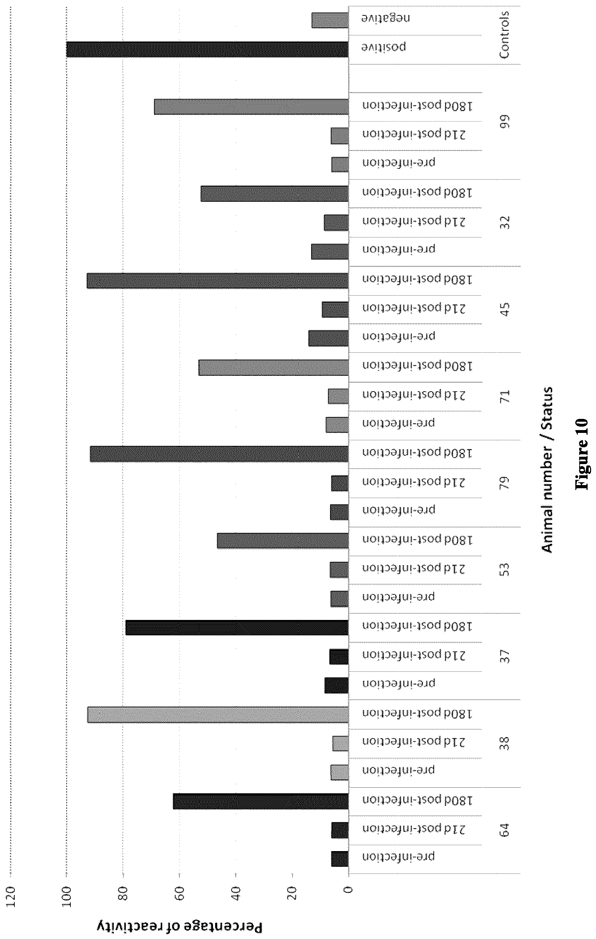

In all the cases, reactivity was only observed in the samples obtained at 180 days (6 months) post-infection. This demonstrates that the present assay can differentiate infected from non-infected animals. The results of this trial are shown in Figure 10, with the positive and negative controls shown at the right of the graph.

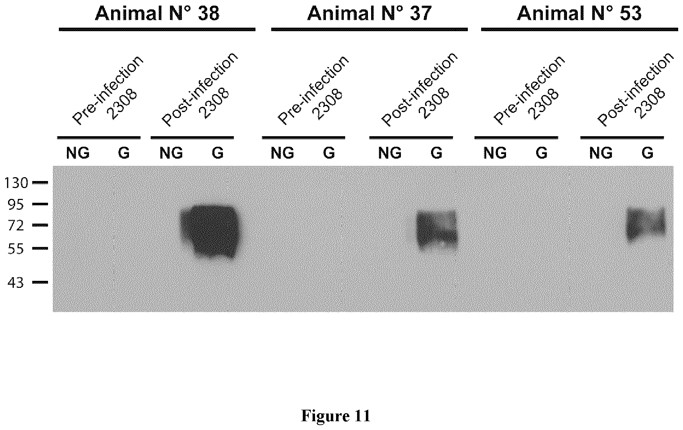

To evaluate the specificity of the reaction, a Western blot analysis was performed using the same bovine samples analyzed above. The results of this assay are shown in Figure 11. Reactivity was only observed against the glycosylated form (G) with the serum samples obtained from infected animals, and not with the non-glycosylated form (NG). These results demonstrate that the reaction is specific for the O-polysaccharide fraction of the glycoconjugate.

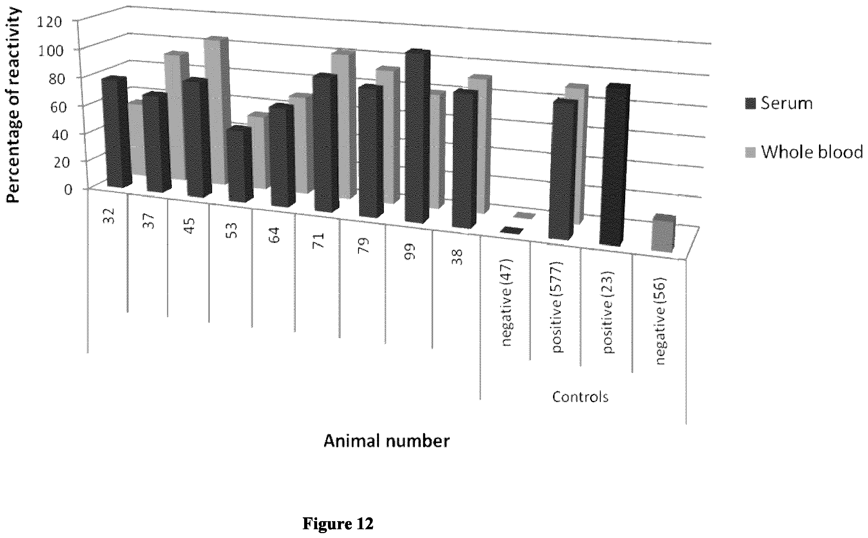

Animals experimentally vaccinated with S19 were challenged with wild type B. *abortus* 2308. In parallel analysis, serum and whole blood samples were obtained and tested 6 months post-infection. The results of this assay are shown in Figure 12. A similar level of reactivity was observed with serum (in dark grey) and blood samples (in light grey) using an equivalent dilution, demonstrating that this assay could be used to diagnose the disease using whole blood samples, making it suitable for field use. On the right, the results obtained with serum and blood samples are shown from a negative and a positive control. (Serum dilution, 1:500; whole blood dilution, 1:250.)

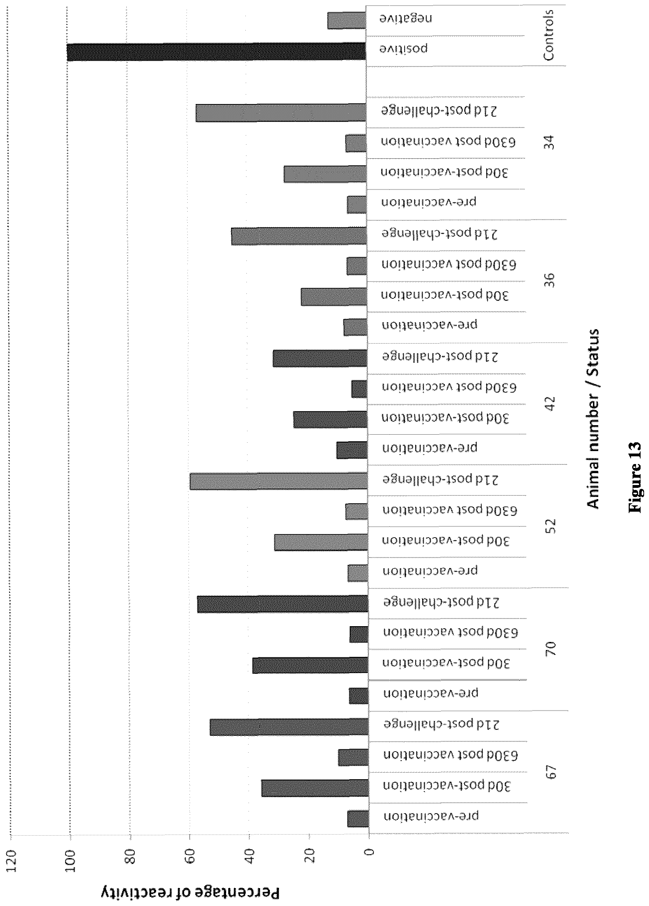

Analysis of serum samples from animals experimentally vaccinated with the smooth vaccine strain S19, was performed using the presently described AcrA-OAg assay. (The S19 vaccine strain is described in: Arenas-Gamboa A.M. et al. Infect Immun. 2009, 77(2):877-84; Crasta O.R. et al. PLoS ONE, 2008, 3(5):1-13; and Klesius P.H. et al. American Journal of Veterinary Research 1978, 39(5):883-886.) Six samples were analyzed for each animal: a pre-vaccination sample, a sample obtained 30 days post-vaccination, samples obtained 395, 520 and 630 days post-vaccination and a sample obtained 21 days post-challenge with a virulent smooth strain. In all the analyzed animals, we have detected reactivity against the antigen at 30 days post-vaccination that then decreased at negative levels. The results of this study are graphically shown in Figure 13. As expected, the reactivity increased after the challenge with the smooth virulent strain. These results demonstrate that the present assay can differentiate vaccinated from infected animals.

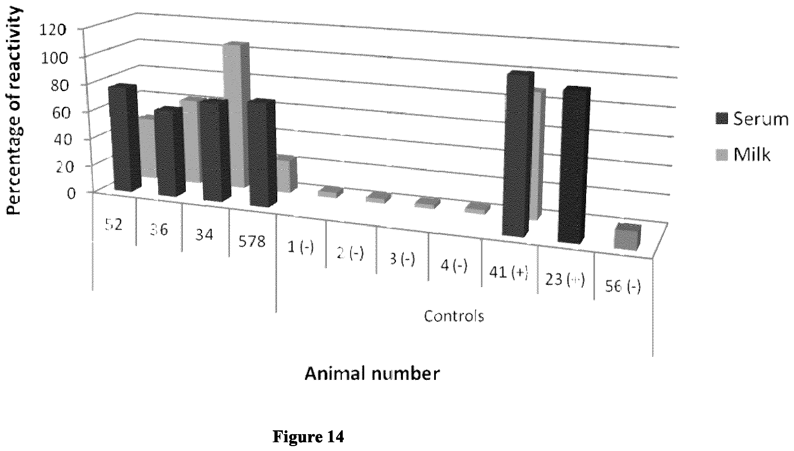

Animals experimentally vaccinated with S19 were challenged with wild type *B. abortus* 2308. In parallel, analysis of serum and milk samples from animals experimentally vaccinated with the smooth vaccine strain S19 was performed. Milk and serum samples were obtained post-delivery or post-abortion at 60 or 90 days post-challenge. The results of this study are shown in Figure 14. In all the analyzed animals, reactivity was observed with serum (in dark grey) and milk samples (in light grey), demonstrating that this assay could be used to diagnose the disease using milk samples (Serum dilution, 1:100; milk dilution, 1:10).

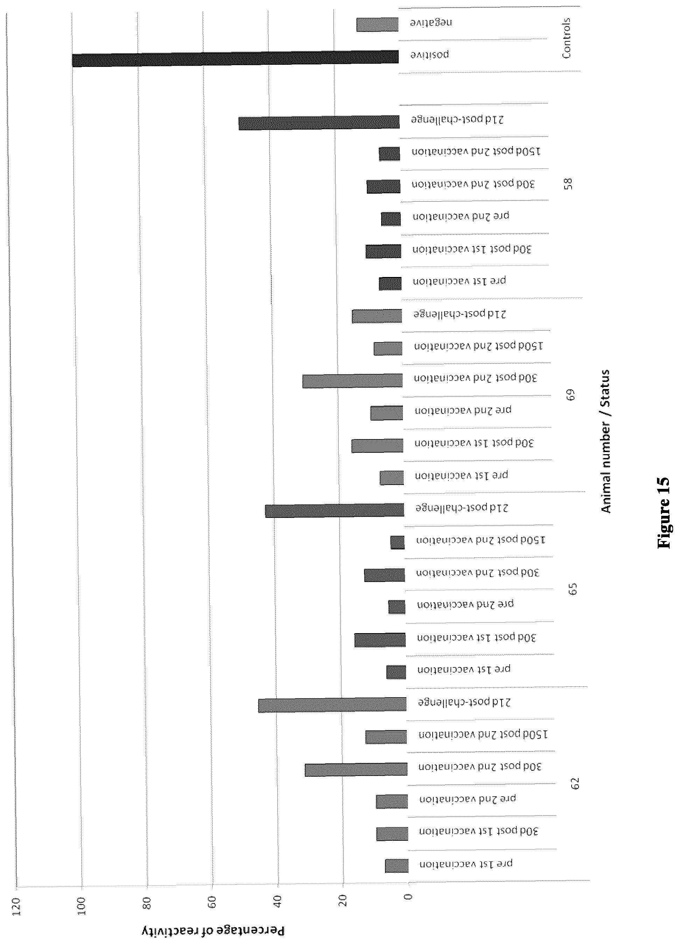

Analysis was also performed using serum samples from animals experimentally vaccinated with the rough vaccine strain Δ*pgm.* (Ugalde J.E. et al. Infect Immun. 2003,71(11): 6264-6269). Six samples were analyzed for each animal: a pre-vaccination sample; a sample obtained 30 days post-first vaccination; a sample obtained pre-second vaccination; samples obtained 30 and 150 days post-second vaccination; and a sample obtained 21 days post-challenge with a virulent smooth strain. The results of this trial are shown in Figure 15. In all the analyzed animals, a low level of reactivity was detected at 30 days post first and second vaccination that decreased to negative levels at 150 days. The reactivity was observed to increase after the challenge with the smooth virulent strain. These results demonstrate that the presently described assay can differentiate vaccinated from infected animals.

## Claims

1. A method for diagnosing a bacterial infection in a subject comprising:
(i) contacting a sample obtained from the subject with a mixture of one or more engineered glycoproteins; and
(ii) detecting first binding complexes formed by binding of the glycoproteins by antibodies present in the sample that are specific for bacterial polysaccharides,
wherein each of said glycoproteins comprises a bacterial antigen covalently attached to a protein via polysaccharyltransferase (PTase)-mediated glycosylation and wherein the presence of binding complexes is indicative of bacterial infection in the subject, wherein the protein is N-glycoprotein AcrA of *Campylobacter jejuni.*

2. The method of claim 1, wherein said one or more glycoproteins are immobilized on a solid medium selected from one or more paramagnetic beads, a surface of one or more microtitre plates, one or more polymer beads, one or more agarose beads, or a membrane.

3. The method of any one of claims 1 or 2, wherein the PTase is PglB or PglL.

4. The method of any one of claims 1 to 3, wherein said detecting step comprises contacting the first binding complexes with a secondary antibody; and detecting second binding complexes formed by binding of the first binding complexes by the secondary antibody.

5. The method of any one of claims 1 to 4, wherein the bacterial antigen comprises an O-antigen of *Yersinia pseudotuberculosis* O9, *Escherichia coli* 0157, or *Brucella abortus* OAg.

6. The method of any one of claims 1 to 4, wherein the bacterial infection comprises an infection of a *Salmonella* sp, *Escherichia coli,* a *Brucella* sp, a *Shigella* sp, *Vibrio cholera, Neisseria meningitidis,* a *Klebsiella* sp, a *Citrobacter* sp, a *Hafnia* sp, a *Proteus* sp, *Haemohilus influenzae, Streptococcus pneumoniae,* a *Staphylococcus* sp, a Group B streptococci, *Yersinia pseudotuberculosis, Campylobacter jejuni, Acinetobacter baumannii* and *Pseudomonas aeruginosa,* such as, an *E. coli* infection or a *Brucella* sp. infection.

7. The method of any one of claims 1 to 6, wherein the sample comprises a bodily fluid selected from serum, plasma, blood, synovial fluid, pharyngeal, nasal/nasal pharyngeal and sinus secretions, saliva, sputum, urine, mucous, feces, chyme, vomit, gastric juices, pancreatic juices, semen/sperm, cerebral spinal fluid, products of lactation or menstruation, egg yolk, amniotic fluid, aqueous humour, vitreous humour, cervical secretions, vaginal fluid/secretions, bone marrow aspirates, pleural fluid, sweat, pus, tears, lymph and bronchial or lung lavage.

8. The method of any one of claims 1 to 7, wherein the subject is a mammal selected from a human or bovine.

9. The method of a claim 4, wherein the secondary antibody is conjugated to a reporter molecule selected from an enzyme or a fluorimetric label.

10. Use of an engineered glycoprotein for an *in vitro* method for diagnosing bacterial infections of any one of claims 1 to 9 comprising an AcrA protein of *Campylobacter jejuni* glycosylated with an O-antigen of *Yersinia pseudotuberculosis* O9 (AcrA-AO9), or an O-antigen of *E. coli* 0157 (AcrAAO157), or an O-antigen of *B. abortus* (AcrA-OAg).

11. Use of an immunoassay kit for an *in vitro* method for diagnosing a bacterial infection of any one of claims 1 to 9 in a subject selected from a human or bovine subject, the kit comprising:
(a) a mixture of one or more engineered glycoproteins, wherein each glycoprotein comprises a bacterial antigen covalently attached to a protein via polysaccharyltransferase (PTase)-mediated glycosylation and wherein the protein is N-glycoprotein AcrA of *Campylobacter jejuni;* and
(b) instructions for contacting the mixture of engineered glycoproteins with a sample of bodily fluid selected from serum, plasma, blood, synovial fluid, pharyngeal, nasal/nasal pharyngeal and sinus secretions, saliva, sputum, urine, mucous, feces, chyme, vomit, gastric juices, pancreatic juices, semen/sperm, cerebral spinal fluid, products of lactation or menstruation, egg yolk, amniotic fluid, aqueous humour, vitreous humour, cervical secretions, vaginal fluid/secretions, bone marrow aspirates, pleural fluid, sweat, pus, tears, lymph and bronchial or lung lavage from the subject and monitoring for formation of binding complexes formed by binding of the glycoproteins by antibodies in the sample that are specific for bacterial polysaccharides.

12. The use of the immunoassay kit of claim 11, wherein the one or more glycoprotein are immobilized on a solid medium, selected from one or more paramagnetic beads, a surface of one or more microtitre plates, one or more polymer beads, one or more agarose beads, or a membrane.

13. The use of the immunoassay kit of any one of claims 11 or 12, additionally comprising a secondary antibody conjugated to a reporter molecule that specifically binds to said antibodies in the sample that are specific for bacterial polysaccharides or to said binding complexes.

14. The use of the immunoassay kit of any one of claims 11 to 13, wherein the bacterial infection is an *E. coli* infection or a *Brucella spp.* infection.

15. The use of the immunoassay kit of any one of claims 11 to 14 , wherein the glycoprotein is an AcrA protein of *Campylobacter jejuni* glycosylated with an O-antigen of *Yersinia pseudotuberculosis* O9 (AcrAAO9), or an O-antigen of *E. coli* 0157 (AcrA-AO157), or an O-antigen of *B. abortus* (AcrA-OAg) as claimed in claim 10.

16. The method of claim 1, wherein the glycoproteins comprise a bacterial polysaccharide covalently attached to a protein, wherein the first binding complexes are formed by binding of the bacterial polysaccharide in the glycoprotein to antibodies present in the sample that are specific for the bacterial polysaccharide, wherein the bacterial polysaccharide and the protein are from different bacterial species.

## Patentansprüche

1. Verfahren zur Diagnose einer Bakterieninfektion in einem Subjekt, umfassend:
(i) das Inkontaktbringen einer von dem Subjekt erhaltenen Probe mit einer Mischung von einem oder mehreren konstruierten Glykoproteinen und
(ii) den Nachweis erster durch die Bindung der Glykoproteine an in der Probe vorhandene, für bakterielle Polysaccharide spezifische Antikörper gebildeter Bindungskomplexe,
wobei die Glykoproteine jeweils ein über Polysaccharyltransferase(PTase)-vermittelte Glykosylierung kovalent an ein Protein gebundenes bakterielles Antigen umfassen und wobei das Vorhandensein von Bindungskomplexen indikativ für eine Bakterieninfektion in dem Subjekt ist, wobei es sich bei dem Protein um das N-Glykoprotein AcrA von *Campylobacter jejuni* handelt.

2. Verfahren nach Anspruch 1, wobei das eine oder die mehreren Glykoproteine auf einem aus einem oder mehreren paramagnetischen Kügelchen, einer Oberfläche einer oder mehrerer Mikrotiterplatten, einem oder mehreren Polymerkügelchen, einem oder mehreren Agarosekügelchen und einer Membran ausgewählten festen Medium immobilisiert sind.

3. Verfahren nach Anspruch 1 oder 2, wobei es sich bei der PTase um PglB oder PglL handelt.

4. Verfahren nach einem der Ansprüche 1 bis 3, wobei der Nachweisschritt das Inkontaktbringen des ersten Bindungskomplexes mit einem sekundären Antikörper und den Nachweis von durch die Bindung der ersten Bindungskomplexe an den sekundären Antikörper gebildeten zweiten Bindungskomplexen umfasst.

5. Verfahren nach einem der Ansprüche 1 bis 4, wobei das bakterielle Antigen ein O-Antigen von *Yersinia pseudotuberculosis* O9, *Escherichia coli* O157 oder *Brucella abortus* OAg umfasst.

6. Verfahren nach einem der Ansprüche 1 bis 4, wobei die Bakterieninfektion eine Infektion mit einer *Salmonella-Sp., Escherichia coli,* einer *Brucella-Sp.,* einer *Shigella-Sp., Vibrio cholera, Neisseria meningitidis,* einer *Klebsiella-Sp.,* einer *Citrobacter-*Sp., einer *Hafnia-Sp.,* einer *Proteus-Sp., Haemophilus influenzae, Streptococcus pneumoniae,* einer *Staphylococcus-Sp.,* einer Gruppe-B-Streptokokke, *Yersinia pseudotuberculosis, Campylobacter jejuni, Acinetobacter baumannii* und *Pseudomonas aeruginosa* wie eine *E.-coli*-Infektion oder eine *Brucella*-Sp.-Infektion umfasst.

7. Verfahren nach einem der Ansprüche 1 bis 6, wobei die Probe eine Körperflüssigkeit ausgewählt aus Serum, Plasma, Blut, Synovialflüssigkeit, Rachen-, Nasen-/Nasenrachen- und Sinussekretionen, Speichel, Auswurf, Urin, Schleim, Fäzes, Chymus, Erbrochenem, Magensäften, Bauchspeicheldrüsensäften, Samen/Sperma, Zerebrospinalflüssigkeit, Laktations- oder Menstruationsprodukten, Eigelb, Fruchtwasser, Kammerwasser, Glaskörper, Gebärmutterhalsabsonderungen, Vaginalflüssigkeit/- absonderungen, Knochenmarkaspiraten, Pleuraflüssigkeit, Schweiß, Eiter, Tränen, Lymphflüssigkeit und Bronchial- oder Lungenlavage umfasst.

8. Verfahren nach einem der Ansprüche 1 bis 7, wobei es sich bei dem Subjekt um ein aus einem Menschen und einem Rind ausgewähltes Säugetier handelt.

9. Verfahren nach Anspruch 4, wobei der sekundäre Antikörper mit einem aus einem Enzym und einem fluorimetrischen Marker ausgewählten Reportermolekül konjugiert ist.

10. Verwendung eines konstruierten Glykoproteins für ein In-vitro-Verfahren zur Diagnose von Bakterieninfektionen nach einem der Ansprüche 1 bis 9, umfassend ein AcrA-Protein von *Campylobacter jejuni,* das mit einem O-Antigen von *Yersinia pseudotuberculosis* O9 (AcrA-AO9) oder einem O-Antigen von *E. coli* 0157 (AcrAAO157) oder einem O-Antigen von *B. abortus* (AcrA-OAg) glykosyliert ist.

11. Verwendung eines Immunassaykits für ein In-vitro-Verfahren zur Diagnose einer Bakterieninfektion nach einem der Ansprüche 1 bis 9 in einem aus einem humanen oder bovinen Subjekt ausgewählten Subjekt, wobei das Kit Folgendes umfasst:
(a) eine Mischung eines oder mehrerer konstruierter Glykoproteine, wobei die Glykoproteine jeweils ein über eine Polysaccharyltransferase(PTase)-vermittelte Glykosylierung kovalent an ein Protein gebundenes bakterielles Antigen umfassen und wobei es sich bei dem Protein um das N-Glykoprotein AcrA von *Campylobacter jejuni* handelt, und
(b) Anweisungen zum Inkontaktbringen der Mischung konstruierter Glykoproteine mit einer Probe einer Körperflüssigkeit ausgewählt aus Serum, Plasma, Blut, Synovialflüssigkeit, Rachen-, Nasen-/Nasenrachen- und Sinussekretionen, Speichel, Auswurf, Urin, Schleim, Fäzes, Chymus, Erbrochenem, Magensäften, Bauchspeicheldrüsensäften, Samen/Sperma, Zerebrospinalflüssigkeit, Laktations- oder Menstruationsprodukten, Eigelb, Fruchtwasser, Kammerwasser, Glaskörper, Gebärmutterhalsabsonderungen, Vaginalflüssigkeit/- absonderungen, Knochenmarkaspiraten, Pleuraflüssigkeit, Schweiß, Eiter, Tränen, Lymphflüssigkeit und Bronchial- oder Lungenlavage von dem Subjekt und zur Prüfung auf die Bildung von durch die Bindung der Glykoproteine an für bakterielle Polysaccharide spezifische Antikörper in der Probe gebildeten Bindungskomplexen.

12. Verwendung des Immunassaykits nach Anspruch 11, wobei das eine oder die mehreren Glykoproteine auf einem aus einem oder mehreren paramagnetischen Kügelchen, einer Oberfläche einer oder mehrerer Mikrotiterplatten, einem oder mehreren Polymerkügelchen, einem oder mehreren Agarosekügelchen und einer Membran ausgewählten festen Medium immobilisiert sind.

13. Verwendung des Immunassaykits nach Anspruch 11 oder 12, zusätzlich umfassend einen sekundären Antikörper konjugiert mit einem Reportermolekül, das spezifisch an die für bakterielle Polysaccharide spezifischen Antikörper in der Probe oder an die Bindungskomplexe bindet.

14. Verwendung des Immunassaykits nach einem der Ansprüche 11 bis 13, wobei es sich bei der Bakterieninfektion um eine *E.-coli*-Infektion oder eine *Brucella-spp.-Infektion* handelt.

15. Verwendung des Immunassaykits nach einem der Ansprüche 11 bis 14, wobei es sich bei dem Glykoprotein um ein AcrA-Protein von *Campylobacter jejuni,* das mit einem O-Antigen von *Yersinia pseudotuberculosis* O9 (AcrA-AO9) oder einem O-Antigen von *E. coli* O157 (AcrA-AO157) oder einem O-Antigen von *B. abortus* (AcrA-OAg) nach Anspruch 10 glykosyliert ist, handelt.

16. Verfahren nach Anspruch 1, wobei die Glykoproteine ein kovalent an ein Protein gebundenes bakterielles Polysaccharid umfassen, wobei die ersten Bindungskomplexe durch die Bindung des bakteriellen Polysaccharids im Glykoprotein an in der Probe vorhandene, für das bakterielle Polysaccharid spezifische Antikörper gebildet werden, wobei das bakterielle Polysaccharid und das Protein von verschiedenen Bakterienspezies stammen.

## Revendications

1. Méthode de diagnostic d'une infection bactérienne chez un sujet, comprenant :
(i) la mise en contact d'un échantillon obtenu à partir du sujet avec un mélange d'une ou plusieurs glycoprotéines manipulées ; et
(ii) la détection de premiers complexes de fixation formés par la fixation des glycoprotéines par des anticorps présents dans l'échantillon qui sont spécifiques pour des polysaccharides bactériens ;
dans laquelle chacune parmi lesdites glycoprotéines comprend un antigène bactérien fixé de manière covalente à une protéine via une glycosylation médiée par une polysaccharide transférase (PTase) et où la présence de complexes de fixation constitue une indication d'une infection bactérienne chez le sujet, où la protéine est une N-glycoprotéine AcrA de *Campylobacter jejuni.*

2. Méthode selon la revendication 1, dans laquelle lesdites une ou plusieurs glycoprotéines sont immobilisées sur un milieu solide choisi parmi une ou plusieurs billes paramagnétiques, une surface d'une ou plusieurs plaques de microtitration, une ou plusieurs billes de polymère, une ou plusieurs billes d'agarose, ou une membrane.

3. Méthode selon l'une quelconque des revendications 1 ou 2, dans laquelle la PTase est Pg1B ou Pg1L.

4. Méthode selon l'une quelconque des revendications 1 à 3, dans laquelle ladite étape de détection comprend la mise en contact des premiers complexes de fixation avec un anticorps secondaire ; et la détection de deuxièmes complexes de fixation formés par la fixation des premiers complexes de fixation par l'anticorps secondaire.

5. Méthode selon l'une quelconque des revendications 1 à 4, dans laquelle l'antigène bactérien comprend un antigène O de *Yersinia pseudotuberculosis* O9, *d'Escherichia coli* O157, ou de *Brucella abortus* OAg.

6. Méthode selon l'une quelconque des revendications 1 à 4, dans laquelle l'infection bactérienne comprend une infection par *Salmonella* sp., *Escherichia coli, Brucella* sp., *Shigella* sp., *Vibrio cholera, Neisseria meningitidis, Klebsiella* sp., *Citrobacter* sp., *Hafnia* sp., *Proteus* sp., *Haemophilus influenzae, Streptococcus pneumoniae, Staphylococcus* sp., des streptocoques du Groupe B, *Yersinia pseudotuberculosis, Campylobacter jejuni, Acinetobacter baumannii* et *Pseudomonas aeruginosa,* telle qu'une infection par *E. coli* ou une infection par *Brucella* sp.

7. Méthode selon l'une quelconque des revendications 1 à 6, dans laquelle l'échantillon comprend un fluide corporel choisi parmi le sérum, le plasma, le sang, le liquide synovial, les sécrétions pharyngiennes, nasales/nasales pharyngiennes et sinusales, la salive, les crachats, l'urine, le mucus, les fèces, le chyme, le vomis, les sucs gastriques, les sucs pancréatiques, le sperme, le liquide céphalorachidien, les produits de la lactation ou de la menstruation, le jaune d'oeuf, le liquide amniotique, l'humeur aqueuse, l'humeur vitreuse, les sécrétions cervicales, le liquide/sécrétions du vagin, les aspirats de la moelle osseuse, le liquide pleural, la sueur, le pus, les larmes, les lavages lymphatiques et bronchiques ou pulmonaires.

8. Méthode selon l'une quelconque des revendications 1 à 7, dans laquelle le sujet est un mammifère choisi parmi un être humain ou un bovin.

9. Méthode selon la revendication 4, dans laquelle l'anticorps secondaire est conjugué à une molécule rapporteuse choisie parmi une enzyme ou un marqueur fluorimétrique.

10. Utilisation d'une glycoprotéine manipulée pour une méthode *in vitro* de diagnostic d'infections bactériennes selon l'une quelconque des revendications 1 à 9, comprenant une protéine AcrA de *Campylobacter jejuni* glycosylée par un antigène O de *Yersinia pseudotuberculosis* O9 (AcrA-AO9), ou un antigène O d'*E*. *coli* O157 (AcrA-AO157), ou un antigène O de *B. abortus* (AcrA-OAg).

11. Utilisation d'un kit d'immunodosage pour une méthode *in vitro* de diagnostic d'une infection bactérienne selon l'une quelconque des revendications 1 à 9 chez un sujet choisi parmi un sujet humain ou bovin, le kit comprenant :
(a) un mélange d'une ou plusieurs glycoprotéines manipulées, où chaque glycoprotéine comprend un antigène bactérien fixé de manière covalente à une protéine via une glycosylation médiée par une polysaccharide transférase (PTase) et où la protéine est une N-glycoprotéine AcrA de *Campylobacter jejuni ;* et
(b) des instructions pour la mise en contact du mélange de glycoprotéines manipulées avec un échantillon de fluide corporel choisi parmi le sérum, le plasma, le sang, le liquide synovial, les sécrétions pharyngiennes, nasales/nasales pharyngiennes et sinusales, la salive, les crachats, l'urine, le mucus, les fèces, le chyme, le vomis, les sucs gastriques, les sucs pancréatiques, le sperme, le liquide céphalorachidien, les produits de la lactation ou de la menstruation, le jaune d'oeuf, le liquide amniotique, l'humeur aqueuse, l'humeur vitreuse, les sécrétions cervicales, le liquide/sécrétions du vagin, les aspirats de la moelle osseuse, le liquide pleural, la sueur, le pus, les larmes, les lavages lymphatiques et bronchiques ou pulmonaires, issu du sujet, et le suivi de la formation de complexes de fixation formés par la fixation des glycoprotéines par des anticorps dans l'échantillon qui sont spécifiques pour des polysaccharides bactériens.

12. Utilisation du kit d'immunodosage selon la revendication 11, dans laquelle les une ou plusieurs glycoprotéines sont immobilisées sur un milieu solide choisi parmi une ou plusieurs billes paramagnétiques, une surface d'une ou plusieurs plaques de microtitration, une ou plusieurs billes de polymère, une ou plusieurs billes d'agarose, ou une membrane.

13. Utilisation du kit d'immunodosage selon l'une quelconque des revendications 11 ou 12, comprenant en outre un anticorps secondaire conjugué à une molécule rapporteuse qui se fixe spécifiquement auxdits anticorps dans l'échantillon qui sont spécifiques pour des polysaccharides bactériens ou auxdits complexes de fixation.

14. Utilisation du kit d'immunodosage selon l'une quelconque des revendications 11 à 13, dans laquelle l'infection bactérienne est une infection par *E. coli* ou une infection par *Brucella* sp.

15. Utilisation du kit d'immunodosage selon l'une quelconque des revendications 11 à 14, dans laquelle la glycoprotéine est une protéine AcrA de *Campylobacter jejuni* glycosylée par un antigène O de *Yersinia pseudotuberculosis* O9 (AcrA-AO9), ou un antigène O d'*E. coli* O157 (AcrA-AO157), ou un antigène O de *B. abortus* (AcrA-OAg) selon la revendication 10.

16. Méthode selon la revendication 1, dans laquelle les glycoprotéines comprennent un polysaccharide bactérien fixé de manière covalente à une protéine, où les premiers complexes de fixation sont formés par la fixation du polysaccharide bactérien dans la glycoprotéine aux anticorps présents dans l'échantillon qui sont spécifiques pour le polysaccharide bactérien, où le polysaccharide bactérien et la protéine sont issus d'espèces bactériennes différentes.
